# EUROPÄISCHE PATENTSCHRIFT

(11) **EP 2 186 522 B1**
(45) Veröffentlichungstag und Bekanntmachung des Hinweises auf die Patenterteilung: **24.01.2018**
(21) Anmeldenummer: 08794081.3
(22) Anmeldetag: 22.07.2008
(51) Int. Cl.: A61K 36/00, B01D 11/02, C11B 9/02

(54) **VERFAHREN ZUR HERSTELLUNG EINES PFLANZLICHEN ZELLSAFTKONZENTRATS**
METHOD FOR PRODUCING VEGETABLE CELL SAP CONCENTRATE
PROCÉDÉ DE PRODUCTION DE SUC CELLULAIRE CONCENTRÉ DE VÉGÉTAUX

(30) Priorität: 07.08.2007 RU 2007130259
(43) Veröffentlichungstag der Anmeldung: 19.05.2010
(73) Patentinhaber: Obschestvo S Ogranichennoi Otvetstvennostiyu "Sibex", Tomsk 634012 (RU); Solagran Limited, Melbourne, VIC 3004 (AU)
(72) Erfinder: KARPITSKIY, Vladimir Ignatovich, Tomsk 634055 (RU); KURGANOV, Aleksandr Kuzmich, Tomsk 634061 (RU)
(74) Vertreter: Vossius & Partner Patentanwälte Rechtsanwälte mbB
(86) Internationale Anmeldenummer: PCT/RU2008/000483
(87) Internationale Veröffentlichungsnummer: WO 2009/031934

(56) Entgegenhaltungen:
- FR-A1- 2 754 464
- RU-C1- 2 067 977
- RU-C2- 2 238 663
- RU-U1- 16 503
- SU-A1- 1 346 111
- US-A- 4 012 194
- US-A- 4 308 200
- US-A- 4 554 170
- US-A1- 2005 257 809

## Beschreibung

Die Erfindung betrifft ein Verfahren zur Herstellung eines Zellsaftkonzentrats aus Pflanzenrohstoffen.

### Gebiet der Technik

Die Erfindung bezieht sich auf die Extraktion biologisch aktiver Substanzen aus Pflanzenrohstoffen und basiert auf der Verwendung von verflüssigtem Kohlendioxid.

Die Erfindung ist in der Pharma-, Parfüm-, Kosmetik- und Lebensmittelindustrie anwendbar.

### Stand der Technik

Die beanspruchte Gruppe von Erfindung dient der Herstellung einer Wasserfraktion aus Pflanzen, die ein praktisch natürliches Zellsaftkonzentrat darstellt, welches aus erntefrischen Pflanzen mit Hilfe von verflüssigtem Kohlendioxid gewonnen wird. Aus dem Stand der Technik wird der Ausdruck "Zellsaft" [1] verwendet.

Es ist bekannt, dass Zellsaft eine Flüssigkeit ist, die vom Zytoplasma einer lebendigen Zelle abgegeben wird und ***deren*** Vakuolen ausfüllt. Die Vakuolen enthalten den Zellsaft, der sich aus Wasser und darin gelösten mineralischen und organischen Substanzen zusammensetzt. Der Zellsaft ist pflanzenfamilien- und auch pflanzenartspezifisch und wird durch die Wachstumsbedingungen, das Alter der Pflanze und deren einzelne Zellen beeinflusst. Die Ansammlung von Zellsaft im Protoplasma ist ein rein physikalischer Prozess, der auch künstlich durchführbar ist.

Aus dem Stand der Technik ist die Gewinnung von Wasserfraktionen (Auszügen) aus Tanne [2], Sanddorn [3], Schneeball [4] usw. bekannt.

Die Gewinnungsverfahren beruhen beispielsweise auf Dampfdestillation biologisch aktiver Substanzen [2], der Extraktion der Pflanzenrohstoffe mittels eines organischen Lösungsmittels und der Verdünnung des gewonnenen Auszugs mit Wasser [3] oder einem Wasser-Ethanol-Gemisch [4] oder auf der Extraktion der Pflanzenrohstoffe unter Druck in zwei Schritten [5].

So wird bei einem bekannten Verfahren zur Gewinnung eines Mittels, das die Widerstandfähigkeit eines Organismus [2] erhöht, ein wässriger Auszug aus Tanne auf folgende Weise erzeugt: Die Rohstoffe werden über 1,5 bis 2 Stunden mit Frischdampf behandelt, das entstehende Dampf-Gas-Gemisch wird abgeführt und der Wasserextrakt wird vom Öl getrennt.

Ein Nachteil dieses Verfahrens besteht darin, dass durch die dabei notwendige hohe Temperatur Vitamine und andere biologisch aktive Substanzen zerstört werden und der gewonnene Auszug nur eine geringe Beständigkeit aufweist.

Aus dem Stand der Technik ist die Verwendung von verflüssigtem Kohlendioxid als Extraktionsmittel bekannt. Bei einem Verfahren zur Extraktion von Pflanzenrohstoffen mit Hilfe von verflüssigten Gase [5] erfolgt die Extraktion der Pflanzenrohstoffe unter Druck in zwei Schritten, wobei eine Durchflussextraktion unter einem Druck, der höher als der Einfülldruck ist, durchgeführt wird und zwischen den Phasen der Druck bis zum Einfülldruck abgebaut wird. Die Durchführungsbedingungen des Verfahrens stellen eine effiziente Tränkung der Rohstoffe sicher, was den Grad der Extraktion der benötigten Komponenten aus den tiefen Schichten der Rohstoffe in einem engeren Zeitraum erhöht.

Bei diesem Verfahren werden zwar Carotinoide, Chlorophyllverbindungen und andere wichtige Komponenten beispielsweise der Lipidfraktion als biologisch aktive Substanzen gewonnen. Das Zellsaftkonzentrat als Zielprodukt fehlt allerdings.

Bekannt ist ein Verfahren zur Gewinnung von Auszügen aus Pflanzenrohstoffen mit Hilfe von verflüssigtem Kohlendioxid [6]. Bei diesem Verfahren wird die Wasserfraktion der Pflanzenrohstoffe jedoch nicht als eigenständiges Produkt abgeschieden, und das Verfahren wird nicht zur Gewinnung der Wasserfraktion, d. h. des Zellsaftkonzentrats, verwendet.

Dem technischen Wesen und angestrebten Ergebnis der Erfindung am nächsten kommt ein Verfahren zur Behandlung von Koniferenrinde [7], das die Zerkleinerung, die Befeuchtung und die Extraktion mit Hilfe von verflüssigtem Kohlendioxid unter Überdruck und bei Raumtemperatur sowie die Trennung des Kohlensäureextrakts in eine Fett- und eine Wasserfraktion umfasst, wobei die Wasserfraktion mit Chloroform extrahiert wird.

Bei diesem Verfahren wird das verflüssigte Kohlendioxid jedoch nicht für die Gewinnung des Zellsafts in nativer Form genutzt, sondern es dient der Extraktion des Wassers, das zu Beginn des Bearbeitungsprozesses für die Befeuchtung der Rinde und die Lösung des aus der Koniferenrinde zu extrahierenden Maltols verwendet wird.

Durch US 4,554,170 ist bekannt, zur Extraktion ätherischer Öle und auch als Kolofonium bekannter Harzkomponenten aus Pflanzenmaterial flüssiges Kohlendioxid unter hohem Druck zu verwenden.

Durch US 4,012,194 ist die Verwendung flüssigen Kohlendioxids in einem geschlossenen Kreislauf zur Extraktion ätherischer Öle bekannt. Eine Abscheidung der Wasserfraktion aus dem geschlossenen Kreislauf ist dabei nicht vorgesehen.

Durch US 2005/0257809 A1 sind ein Verfahren und eine Vorrichtung zur Trennung von öl-, Fett- und Gleitstoffrückständen sowie von Partikeln von Feststoffen vermittels flüssigen Kohlendioxids zum Zwecke einer getrennten Entsorgung dieser Substanzen bekannt. Das flüssige Kohlendioxid löst zunächst die genannten Substanzen und wird anschließend hiervon durch Verdampfen getrennt. Das verdampfte Kohlendioxid wird anschließend verdichtet und in einem Kreislauf wieder verwendet. Die gelösten Substanzen werden dem Kreislauf gemeinsam entnommen.

Die durchgeführte Analyse hat gezeigt, dass aus dem Stand der Technik die Verwendung von verflüssigtem Kohlendioxid zur Gewinnung von pflanzlichem Zellsaft nicht bekannt ist.

### Offenbarung der Erfindung

Die Aufgabe der Erfindung besteht darin, ein Verfahren zur Gewinnung von pflanzlichem Zellsaftkonzentrat bereitzustellen.

Die gestellte Aufgabe wird mit einem Verfahren nach Anspruch 1 gelöst.

Das technische Ergebnis besteht in der Bereitstellung einer Möglichkeit zur Gewinnung eines Zellsaftkonzentrats. Dabei werden die Ausbeute und die Qualität der nutzbaren Komponenten der Pflanzenrohstoffe dadurch erhöht, dass das Zellsaftkonzentrat in nativer Form gewonnen wird. Dabei wird flüssiges Kohlendioxid verwendet.

Die gestellte Aufgabe wird dadurch gelöst, dass
bei einem bekannten Verfahren, das die Extraktion zerkleinerter Rohstoffe mit Hilfe von verflüssigtem Kohlendioxid bei Raumtemperatur und die Abscheidung der Ölfraktion umfasst, die Extraktion unter einem großen Durchsatz des verflüssigten Kohlendioxids durchgeführt wird, ausgehend von einem Verhältnis von mindestens 11 Kohlendioxid auf 1 g gewonnenen pflanzlichen Zellsaft, wobei das Zellsaftkonzentrat durch Separieren der Ölfraktion von der Wasserfraktion gewonnen wird.

Das technische Ergebnis wird im Wesentlichen dadurch erreicht, dass als Mittel zur Gewinnung des pflanzlichen Zellsaftkonzentrats verflüssigtes Kohlendioxid verwendet wird. Die Extraktion der Rohstoffe erfolgt bevorzugt mit Hilfe von verflüssigtem Kohlendioxid in einem Extraktor durch Zugabe des verflüssigten Kohlendioxids aus einem Sammelbehälter, der in einer Höhe von mindestens 8 bis 12 m über dem Extraktor angeordnet ist.

Die Extraktion der Rohstoffe mit Hilfe von verflüssigtem Kohlendioxid kann auch durch Zugabe des verflüssigten Kohlendioxids mittels einer Dosierpumpe erfolgen.

Optimal ist die Verwendung erntefrischer Pflanzenrohstoffe für die Extraktion.

Die Extraktion mit Hilfe von verflüssigtem Kohlendioxid gewährleistet eine selektive und relativ umfassende Abscheidung des Zellsafts in Form der Wasserfraktion aus den Pflanzenrohstoffen, da das verflüssigte Kohlendioxid sowohl das Wasser als auch die wasserlöslichen Verbindungen löst. Dies ist dadurch zu erklären, dass die Löslichkeit von Wasser in flüssigem Kohlendioxid 0,1 % beträgt, d. h. für die Gewinnung von 1 Kilo (oder Liter) Zellsaft müssen 1.000 kg verflüssigtes Kohlendioxid durch die Pflanzenrohstoffe geleitet werden. Zur Abscheidung der Ölfraktion in einer Menge von 1 kg reichen dagegen einige Dutzend Liter verflüssigten Kohlendioxids. Zur Abscheidung des Zellsafts in Form der Wasserfraktion muss daher eine ausreichend hohe Menge an verflüssigtem Kohlendioxid durch den Durchflussextraktor geleitet werden. In herkömmlichen Anlagen beträgt die Installationshöhe des Sammelbehälters für verflüssigtes Kohlendioxid über dem Extraktor 0,5 bis 1 m, weshalb der Durchsatz zwar zur Abscheidung der Ölfraktion, nicht jedoch zur Ausscheidung der Wasserfraktion ausreichend ist. Gemäß der vorliegenden Erfindung kann der Durchsatz des Kohlendioxids durch die Höhe der Flüssigkeitssäule erreicht werden. Baulich wird dies mit der Schaffung eines Druckunterschiedes durch Anheben des Sammelbehälters des verflüssigten Kohlendioxids auf eine Höhe von 8 bis 12 m über dem Extraktor erreicht. Diese Aufgabe kann auch durch die Verwendung einer Hochdruckpumpe gelöst werden. Der Extraktionsprozess erfolgt bei Raumtemperatur, was keine destruktive Veränderungen der chemischen Verbindungen zur Folge hat, sondern dazu führt, dass der mit Hilfe des verflüssigten Kohlendioxids unter Druck aus den erntefrischen Pflanzenrohstoffen extrahierte Zellsaft ohne Wärmebehandlung in nativer Form bereitgestellt wird.

Bei der beanspruchten Gruppe von Erfindungen wird bei keinem der Extraktionsschritte Wasser zur Gewinnung der Wasserfraktion der Pflanzenrohstoffe hinzugefügt. Somit handelt es sich bei dem wässrigen Auszug um den natürlichen Zellsaft einer der Extraktion unterzogenen Pflanze, der die Gesamtheit der biologisch aktiven Substanzen enthält.

Die Verwendung von verflüssigtem Kohlendioxid zur Durchführung des Extraktionsprozesses ist bekannt, seine Verwendung als Mittel zur Gewinnung eines Zellsaftkonzentrats ist aus dem Stand der Technik dagegen nicht bekannt. Allgemein bekannt ist die Abscheidung ätherischer Öle und anderer fettlöslicher Pflanzenkomponenten aus Pflanzenrohstoffen mit Hilfe von verflüssigtem Kohlendioxid. Allerdings lösen sich Wasser und wasserlösliche Pflanzenstoffe in verflüssigtem Kohlendioxid nur in geringen Mengen [Wasser (0,1 %)]. Am Ende des Extraktionsprozesses und der Entfernung des Kohlendioxids mittels Druckablasses werden das Wasser und die wasserlöslichen Stoffe von der Ölfraktion getrennt und als separate Phase abgeschieden. Da bei den bekannten technischen Lösungen die Menge des extrahierten Wassers und der wasserlöslichen Komponenten verschwindend gering ist und das Zielprodukt in der Regel die Ölfraktion ist, finden sich in der wissenschaftlich-technischen Patentliteratur auch keine Angaben über Wasserfraktionen, die mit Hilfe von verflüssigtem Kohlendioxid extrahiert werden. Folglich erfüllt die beanspruchte Gruppe von Erfindungen die Kriterien "Neuheit" und "Erfindungshöhe".

### Ausführungsbeispiele der Erfindung

Das Verfahren zur Gewinnung eines wässrigen Auszugs aus Tanne wird folgendermaßen ausgeführt:

Die zerkleinerten, vorzugsweise erntefrischen Pflanzenstoffe werden mit Hilfe von Kohlendioxid bei Raumtemperatur und unter einem Druck von 64 ± 6 atm (eine Extraktionszeit von 3 Stunden ist ausreichend) in einem Durchflussextraktor mit einem Durchsatz des verflüssigten Kohlendioxids, der durch das Anheben des Sammelbehälters mit dem verflüssigten Kohlendioxid auf eine Höhe von 8 bis 12 m über dem Extraktor erhöht ist, vollständig extrahiert. Die Erhöhung des Durchsatzes des verflüssigten Kohlendioxids durch die extrahierten Pflanzenrohstoffe wird durch die Vergrößerung der Säule des verflüssigten Kohlendioxids über dem Extraktor sichergestellt, wodurch der Druck der Flüssigkeitssäule auf die Rohstoffe proportional zunimmt. Experimentelle Untersuchungen haben gezeigt, dass bei einer Vergrößerung der Flüssigkeitssäule von 0,5 bis 1 m, wie sie üblicherweise in herkömmlichen Anlagen und im Prototyp (zur Abscheidung anderer Zielprodukte, beispielsweise einer Ölfraktion) verwendet wird, auf 8 bis 12 m der Durchsatz um das 8- bis 24-fache steigt und dass der Druck zunimmt, was zu einer proportionalen Erhöhung der Menge des extrahierten Wassers und der wasserlöslichen Stoffe führt.

Die Vergrößerung des Durchsatzes des verflüssigten Kohlendioxids durch die extrahierten Pflanzenrohstoffe kann auch durch die Verwendung einer Dosierpumpe erreicht werden. Nach der Gewinnung des Kohlendioxidextrakts wird diesem das Kohlendioxid entzogen und ein Gesamtextrakt gewonnen, der sich durch Absetzenlassen oder Zentrifugieren in zwei Fraktionen - eine Öl- und eine Wasserfraktion - trennt. Die Ausbeute der Wasserfraktion des Kohlendioxidextrakts der Pflanze bildet den Zellsaft mit einer hohen Konzentration an biologisch aktiven Substanzen. Die Verwendung des vorgeschlagenen Verfahrens ermöglicht es, einen Extrakt wasserlöslicher pflanzlicher Verbindungen, d. h. Zellsaft, zu gewinnen, ohne deren Bioaktivität zu beeinträchtigen. Dies lässt sich dadurch erklären, dass die Extraktion von Pflanzenrohstoffen mit Hilfe von Kohlendioxid nicht zu destruktiven Veränderungen der organischen Moleküle führt, da der Prozess bei Raumtemperatur durchgeführt wird und sich Kohlendioxid als Inertgas etabliert hat.

Die Konzentration des Zellsafts mit Hilfe von verflüssigtem Kohlendioxid lässt sich folgenderweise erklären. Betrachten wir den Prozess der Zellsaftkonzentration bei der Kohlensäureextraktion am Beispiel von Tannennadeln, die als meist verwendeter Rohstoff für die Herstellung von Tannenextrakt mit Hilfe von Kohlendioxid gelten. Diese Erläuterungen sind auch auf jede andere Pflanze anwendbar. 1.000 kg erntefrischer Tannennadeln enthalten ca. 600 kg Wasser (die Feuchtigkeit von erntefrischen Tannennadeln beträgt 55 bis 65 %). Experimentelle Daten haben gezeigt, dass diese Rohstoffe nur etwa 0,6 kg an wasserlöslichen biologisch aktiven Substanzen, insbesondere Ascorbinsäure, Maltol, organische Salze u. a., enthalten. Nimmt man hypothetisch an, dass diese Verbindungen zusammen mit dem Wasser vollständig extrahiert werden, so wird der Zellsaft der Pflanze gewonnen (600 kg mit einem Trockenrückstand von 0,1 %). Experimente zum beanspruchten Verfahren und Mittel haben gezeigt, dass nach dem Durchleiten von 1.000 kg verflüssigten Kohlendioxids 1 kg Wasser und 0,6 kg wasserlöslicher Verbindungen gewonnen werden. Das Verfahren ermöglicht es also, ein pflanzliches Zellsaftkonzentrat zu gewinnen, das praktisch alle wasserlöslichen Pflanzenkomponenten enthält. Die durchgeführte Analyse hat das praktisch vollständige Fehlen von Ascorbinsäure und Maltol in den extrahierten Rohstoffen ergeben, was von der vollständigen Extraktion der wasserlöslichen Komponenten zeugt und dem Inhalt des Zellsafts entspricht. Die experimentellen Untersuchungen haben ferner gezeigt, dass das gewonnene Zellsaftkonzentrat 3 % Trockenrückstand enthält, was von einer Erhöhung der Konzentration der gelösten Stoffe im Zellsaftkonzentrat um das 30-fache zeugt. Bei weiterer Fortsetzung der Extraktion wird zudem fast nur Wasser gewonnen (1 kg pro 1.000 kg Kohlensäure). Um das gesamte Wasser aus 1.000 kg Tannennadeln abzuscheiden, müsste man 600.000 kg verflüssigtes Kohlendioxid durch die extrahierten Rohstoffe leiten, was weder praktikabel noch notwendig ist.

Der Prozess der Extraktion mit Hilfe von Kohlendioxid wird nicht oder nur in geringem Maß von der Temperatur und vom Druck beeinflusst. Der Druck des flüssigen Kohlendioxids hängt eindeutig von der Temperatur ab, d. h. bei der Erhöhung der Temperatur steigt proportional auch der Druck. Bei einer Raumtemperatur, die in der Regel im Bereich von 15 bis 30 °C liegt, beträgt der Druck 40 bis 74 atm.

Die praktische Umsetzung des Verfahrens wird anhand folgender Beispiele erörtert.

### Beispiel 1 (am nächsten kommende Erfindung)

Erntefrische und zerkleinerte Nadeln der sibirischen Tanne (58 kg) werden bei einer Temperatur von 100 bis 180 °C und unter einem Druck von 1 bis 2 atm über 10 bis 24 Stunden dampfdestilliert. Die Ausbeute an ätherischem Öl beträgt 1,45 kg (2,5 % der Ausgangsmasse an Tannennadeln). Die Ausbeute der Wasserfraktion beträgt 45 kg. Die Wasserfraktion enthält 0,009 % Trockenrückstände.

### Beispiel 2

Getrocknete und auf Partikel mit einer Größe von maximal 5 mm zerkleinerte Nadeln der sibirischen Tanne (58 kg), werden in einem Durchflussextraktor mit einer Flüssigkeitssäule von 0,5 m Höhe mit Hilfe von verflüssigtem Kohlendioxid bei einer Temperatur von 20 °C und unter einem Druck von 64 atm über 3 Stunden extrahiert. Die Ausbeute beträgt 1,8 kg (3,1 % der Ausgangsmasse an Tannennadeln). Die Wasserfraktion fehlt.

### Beispiel 3 (Prototyp)

Erntefrische und zerkleinerte Nadeln der sibirischen Tanne (58 kg) werden in einem einfachen Durchflussextraktor mit Hilfe von verflüssigtem Kohlendioxid bei einer Temperatur 20 °C und unter einem Druck von 64 atm über 3 bis 24 Stunden extrahiert. Die Ausbeute der Ölfraktion beträgt 1,2 kg (2,1 % der Ausgangsmasse an Tannennadeln). Die Wasserfraktion in einer Menge von 80 g ergibt eine Emulsion mit der Ölfraktion, die als Ausschuss anzusehen ist und nach langem Absetzenlassen oder Zentrifugieren in Form eines feuchten festen Produkts, das der Abscheidung von Maltol dient, verworfen wird. Die Wassermenge wurde durch ein Wiegeverfahren beim Trocknen der feuchten festen "Maltolmasse bestimmt. Laut Berechnung beträgt der Gesamtdurchsatz des verflüssigten Kohlendioxids 80 Liter.

### Beispiel 4

Erntefrische und zerkleinerte Nadeln der sibirischen Tanne (58 kg) werden in einem Durchflussextraktor mit Hilfe von verflüssigtem Kohlendioxid bei einem hohen Durchsatz des verflüssigten Kohlendioxids durch das Anheben des Sammelbehälters des verflüssigten Kohlendioxids auf eine Höhe von 10 m über dem Extraktor bei einer Temperatur von 20° C und unter einem Druck von 64 atm über 3 Stunden extrahiert. Die Ausbeute an Gesamtextrakt beträgt 2,3 kg (4,0 % der Ausgangsmasse an Tannennadeln). Nach dem Absetzenlassen oder Zentrifugieren werden zwei Fraktionen - eine Ölfraktion von 1,4 kg (4 %) und eine Wasserfraktion von0,9 kg (1,6%) - gewonnen. Laut Berechnung betrug der Gesamtdurchsatz des verflüssigten Kohlendioxids 900 Liter.

Das gemäß Beispiel 4 gewonnene Produkt hat das Aussehen einer dunkelroten Lösung und ist in Wasser und Alkohol lösbar. Der Trockenrückstand beträgt 2,9 %, d. h. hinsichtlich des Inhalts der gelösten Stoffe übertrifft dieses die bekannten Verfahren, bei denen der Trockenrückstand nicht mehr als 0,016 [%] beträgt (aus der Quelle [2]), etwa um das 300-fache. Der Ascorbinsäuregehalt beträgt 750 mg/kg.

### Beispiel 5

Erntefrische und zerkleinerte Nadeln der sibirischen Tanne (58 kg) werden im Durchflussextraktor mit Hilfe von verflüssigtem Kohlendioxid bei einem hohen Durchsatz des verflüssigten Kohlendioxids durch das Anheben des Sammelbehälters des verflüssigten Kohlendioxids auf eine Höhe von 8 m über dem Extraktor bei einer Temperatur von 20 °C und unter einem Druck von 64 atm über 3 Stunden extrahiert. Die Ausbeute an Gesamtextrakt beträgt 2,1 kg (3,6 % der Ausgangsmasse an Tannennadeln). Nach dem Absetzenlassen oder Zentrifugieren werden zwei Fraktionen - eine Ölfraktion von 1,4 kg (2,4 %) und eine Wasserfraktion von 0,7 (1,6 %) - gewonnen. Laut Berechnung betrug der Gesamtdurchsatz des verflüssigten Kohlendioxids 700 Liter.

### Beispiel 6

Erntefrische und zerkleinerte Nadeln der sibirischen Tanne (58 kg) werden im Durchflussextraktor mit Hilfe von verflüssigtem Kohlendioxid bei einem hohen Durchsatz des verflüssigten Kohlendioxids durch das Anheben des Sammelbehälters des verflüssigten Kohlendioxids auf eine Höhe von 12 m über dem Extraktor bei einer Temperatur von 20 °C und einem Druck von 64 atm über 3 Stunden extrahiert. Die Ausbeute an Gesamtextrakt beträgt 2,5 kg (4,3 % der Ausgangsmasse an Tannennadeln). Nach dem Absetzenlassen oder Zentrifugieren werden zwei Fraktionen - eine Ölfraktion von 1,4 kg (2,4 %) und eine Wasserfraktion von 1,1 kg (1,9%) - gewonnen. Laut Berechnung betrug der Gesamtdurchsatz des verflüssigten Kohlendioxids 1.100 Liter.

### Beispiel 7

Erntefrisches Birkenlaub in einer Menge von 2,7 kg wird behandelt, wie in Beispiel 4 beschrieben. Die Ausbeute an Gesamtextrakt beträgt 52 g. Nach dem Zentrifugieren werden zwei Fraktionen - eine Ölfraktion von 21 g und eine Wasserfraktion von 31 g - gewonnen.

### Beispiel 8

Erntefrische Brennnesselblätter in einer Menge von 9,2 kg werden behandelt, wie in Beispiel 4 beschrieben. Die Ausbeute an Gesamtextrakt beträgt 802 g. Nach dem Zentrifugieren werden zwei Fraktionen - eine Ölfraktion von 482 g und eine Wasserfraktion von 320 g - gewonnen.

### Beispiel 9

Erntefisches Preiselbeerlaub in einer Menge von 6,7 kg wird behandelt, wie in Beispiel 4 beschrieben. Die Ausbeute an Gesamtextrakt beträgt 680 g. Nach dem Zentrifugieren werden zwei Fraktionen - eine Ölfraktion von 8 g und eine Wasserfraktion von 672 g - gewonnen.

### Beispiel 10

Erntefrische Johannisbeerblätter in einer Menge von 8,2 kg werden behandelt, wie in Beispiel 4 beschrieben. Die Ausbeute an Gesamtextrakt beträgt 97 g. Nach dem Zentrifugieren werden zwei Fraktionen - eine Ölfraktion von 74 g und eine Wasserfraktion von 23 g - gewonnen.

### Beispiel 11

Rückstände von Sanddornbeeren in einer Menge von 9,2 kg werden behandelt, wie in Beispiel 4 beschrieben. Die Ausbeute an Gesamtextrakt beträgt 286 g. Nach dem Zentrifugieren werden zwei Fraktionen - eine Ölfraktion von 3 g und Wasserfraktion von 283 g - gewonnen.

### Beispiel 12

Rückstände von Beeren des Schneeballs in einer Menge von 6,87 kg werden behandelt, wie in Beispiel 4 beschrieben. Die Ausbeute an Gesamtextrakt beträgt 135 g. Nach dem Zentrifugieren werden zwei Fraktionen - eine Ölfraktion von 92 g und eine Wasserfraktion von 42 g - gewonnen.

### Beispiel 13

Erntefrische und zerkleinerte Nadeln der sibirischen Tanne (58 kg) werden im Durchflussextraktor mit Hilfe von verflüssigtem Kohlendioxid bei einem hohen Durchsatz des verflüssigten Kohlendioxids durch die Verwendung einer Hochdruckpumpe für Flüssigkeiten mit einer Leistung von 100 I/Stunde bei einer Temperatur von 20 °C und unter einem Druck von 64 atm über 3 Stunden extrahiert. Die Ausbeute an Gesamtextrakt beträgt 1,7 kg (**2,9 %** der Ausgangmasse an Tannennadeln). Nach dem Absetzenlassen oder Zentrifugieren werden zwei Fraktionen - eine Ölfraktion von 1,4 kg (4 %) und eine Wasserfraktion von 0,3 (0,5 %) - gewonnen. Laut Berechnung betrug der Gesamtdurchsatz des verflüssigten Kohlendioxids 300 Liter.

Das gemäß Beispiel 13 gewonnene Produkt hat das Aussehen einer dunkelroten Lösung und ist in Wasser und Alkohol lösbar. Der Trockenrückstand beträgt 3,0 %.

### Beispiel 14

Erntefrische Nadeln der sibirischen Tanne (58 kg), die auf eine Partikelgröße von maximal 5 mm zerkleinert wurden, werden im Durchflussextraktor mit Hilfe von verflüssigtem Kohlendioxid bei einem hohen Durchsatz des verflüssigten Kohlendioxids durch Verwendung einer Hochdruckpumpe für Flüssigkeiten mit einer Leistung von 300 I/Stunde bei einer Temperatur von 20 °C und unter einem Druck von 64 atm über 3 Stunden extrahiert. Die Ausbeute an Gesamtextrakt beträgt 2,3 kg (4,0 % der Ausgangsmasse an Tannennadeln). Nach dem Absetzenlassen oder Zentrifugieren werden zwei Fraktionen - eine Ölfraktion von 1,4 kg (4 %) und eine Wasserfraktion von 0,9 kg (1,6 %) - gewonnen. Laut Berechnung betrug der Gesamtdurchsatz des verflüssigten Kohlendioxids 900 Liter.

Das gemäß Beispiel 14 gewonnene Produkt hat das Aussehen einer dunkelroten Lösung und ist in Wasser und Alkohol lösbar. Der Trockenrückstand beträgt 2,9 %. Die durchgeführten Experimente und anschließende Erprobung und gewerbliche Nutzung des Verfahrens zur Gewinnung von Zellsaftkonzentrat, das auf der Verwendung einer großen Menge an verflüssigtem Kohlendioxid basiert, haben eine hohe Effizienz gezeigt. Hinsichtlich des Trockenrückstands, in dem alle wirksamen Substanzen enthalten sind, übertrifft die Wasserfraktion des mit Hilfe von Kohlendioxid gewonnenen Pflanzenrohstoffextrakts, d. h. des Zellsaftkonzentrats, alle bekannten Analoga, die mittels anderer Verfahren zur Gewinnung von Wasserfraktionen erhalten werden.

### Gewerbliche Anwendbarkeit

Die Verwendung von verflüssigter Kohlensäure nach dem beanspruchten Verfahren hat es ermöglicht, die Wasserfraktion von Pflanzen zu gewinnen, die den Zellsaft der Pflanze mit den darin gelösten biologisch aktiven Substanzen in konzentrierter Form, d. h. ein Zellsaftkonzentrat, darstellt. Dabei enthält der Zellsaft wertvolle natürliche Substanzen in nativer (lebendiger) Form und in maximaler, konzentrierter Menge. Die Erfindung ist in der Pharma-, Parfüm-, Kosmetik- und Lebensmittelindustrie anwendbar.

### Literatur

[1] Bol'šaja medicinskaja ėnciklopedija [deutsch: Große medizinische Enzyklopädie], 1973, Band 12, Moskau, Verlag Sovetskaja ėnciklopedija, S. 890.
[2] Patent der Russischen Föderation Nr. 2061491, IPC 6 A61K35/78, veröffentlicht am 10.06.1996.
[3] Patent der Russischen Föderation Nr. 2125459, IPC 6 A61K35/78, veröffentlicht am 27.01.1999.
[4] Patent der Russischen Föderation Nr. 2220614, IPC 7 A23L1/30, A61K35/78, veröffentlicht am 10.01.2004.
[5] Patent der Russischen Föderation Nr. 2039586, IPC 6 B01D11/02, A61K35/78 veröffentlicht am 20.07.1995
[6] Patent der Russischen Föderation Nr. 2259991, IPC 7 C07C33/02, veröffentlicht am 2259991 [*sic*]*.*
[7] Patent der Russischen Föderation Nr. 2067977, IPC 6 C07D309/40, veröffentlicht am 20.10.1996.

## Patentansprüche

1. Verfahren zur Herstellung des Zellsaftkonzentrats der Pflanzen, das eine Extraktion der zerkleinerten Rohstoffe mit Hilfe von verflüssigtem Kohlendioxid bei Raumtemperatur und Ausscheidung der Ölfraktion beinhaltet, **dadurch gekennzeichnet,**
**dass** die Extraktion bei einem großen Durchfluss des verflüssigten Kohlendioxids ausgehend von einem Verhältnis von mindestens 11 Kohlendioxid für 1 g gewonnenes Zellsaftkonzentrat der Pflanzen durchgeführt wird, wobei das Zellsaftkonzentrat in Form einer Wasserfraktion gewonnen wird, von der die Ölfraktion getrennt ist.

2. Verfahren nach Anspruch 1, **dadurch gekennzeichnet,**
**dass** die Extraktion der Rohstoffe mit Hilfe des verflüssigten Kohlendioxids in einem Extraktor durch Zufuhr des verflüssigten Kohlendioxids aus einem Speicher durchgeführt wird, der auf einer Höhe von mindestens 8 bis 12 m über dem Extraktor installiert wird.

3. Verfahren nach Anspruch 1, **dadurch gekennzeichnet,**
**dass** die Extraktion der Rohstoffe durch Zufuhr des verflüssigten Kohlendioxids mittels einer Dosierpumpe durchgeführt wird.

4. Verfahren nach Anspruch 1, **dadurch gekennzeichnet,**
**dass** für die Extraktion frisch geerntete Pflanzenrohstoffe verwendet werden.

## Claims

1. Method for producing plant cell sap concentrate, which includes an extraction of the chopped raw materials with the aid of liquefied carbon dioxide at room temperature and separation of the oil fraction, **characterized in that**
the extraction is carried out at a high flow rate of the liquefied carbon dioxide starting from a ratio of at least 1 litre of carbon dioxide per 1 gram of obtained plant cell sap concentrate, wherein the cell sap concentrate is obtained in the form of a water fraction from which the oil fraction is separated.

2. Method according to Claim 1, **characterized in that**
the extraction of the raw materials with the aid of the liquefied carbon dioxide is carried out in an extractor by supplying the liquefied carbon dioxide from a storage tank which is installed at a height of at least 8 to 12 m above the extractor.

3. Method according to Claim 1, **characterized in that**
the extraction of the raw materials by supplying the liquefied carbon dioxide is carried out by means of a metering pump.

4. Method according to Claim 1, **characterized in that** freshly collected plant raw materials are used for the extraction.

## Revendications

1. Procédé de fabrication d'un suc cellulaire concentré de végétaux, qui comprend une extraction des matières premières broyées à l'aide de dioxyde de carbone liquéfié à température ambiante et une séparation de la fraction huileuse, **caractérisé en ce que**
l'extraction est réalisée à un débit important du dioxyde de carbone liquéfié en partant d'un rapport d'au moins 1 1 de dioxyde de carbone pour 1 g de suc cellulaire concentré de végétaux obtenu, le suc cellulaire concentré étant obtenu sous la forme d'une fraction aqueuse de laquelle la fraction huileuse est séparée.

2. Procédé selon la revendication 1, **caractérisé en ce que** l'extraction des matières premières est réalisée à l'aide du dioxyde de carbone liquéfié dans un extracteur par introduction du dioxyde de carbone liquéfié à partir d'un stockage, qui est installé à une hauteur d'au moins 8 à 12 m au-dessus de l'extracteur.

3. Procédé selon la revendication 1, **caractérisé en ce que** l'extraction des matières premières est réalisée par introduction du dioxyde de carbone liquéfié au moyen d'une pompe de dosage.

4. Procédé selon la revendication 1, **caractérisé en ce que** des matières premières végétales fraîchement récoltées sont utilisées pour l'extraction.
